# EUROPEAN PATENT APPLICATION

(11) **EP 1 864 631 A1**
(43) Date of publication of application: **12.12.2007**
(21) Application number: 07301007.6
(22) Date of filing: 30.04.2007
(51) Int. Cl.: A61F 5/41, A61F 6/02

(54) **Insertion assisting device**

(30) Priority: 05.06.2006 JP 2006182483
(71) Applicant: Kohritsu Shoji Co., Ltd., Kanda Chiyoda-ku Tokyo (JP)
(72) Inventor: Shioiri, Kazuhiro, c/o KOHRITSU SHOJI CO., LTD., Tokyo (JP)
(74) Representative: Catherine, Alain

(57) **Abstract**

The insertion assisting device allows a penis, though having a decreased erectile function, to be inserted smoothly into a vagina and dissolves within a few minutes of insertion, so that the penis can be kept inserted in the vagina in a natural state of insertion. Thus, a good physical contact between spouses and hence a desirable marital relationship can be maintained. The insertion assisting device (10) includes a cylindrical member (14) for covering a penis, the cylindrical member including a front portion (12) having a round tip (12a) at which the front portion closes and tapering in a curve toward the round tip and a cylindrical portion having a circular opening end (14a). The cylindrical member is a hard capsule formed from a soluble material that dissolves in body fluid existing inside the vagina.

## Description

The entire contents of literatures cited in this specification are incorporated herein by reference.

### BACKGROUND OF THE INVENTION

The present invention relates to an insertion assisting device for enabling a penis, though having a decreased erectile function, to be inserted into a vagina.

Attempts have been heretofore made to help a penis, of which the erectile function has decreased due to psychological or physical causes, or because of age, or for other various reasons, to recover its erectile function physically or mimetically, some of them proposing devices that are attached to a penis.

A decreased erectile function of a penis is a disorder defined as ED (erectile dysfunction) or "a condition inhibiting a satisfactory sexual intercourse because of the inability to achieve sufficient erection or maintain an erection at the time of sexual intercourse," and may develop in a number of males. However, the disorder has been long suffering prejudice as it is referred to as "impotent (sexually disabled)" as though a male with ED lacked an ability essential to a human.

Against such backdrop, the use of devices intended to remedy decreased erectile function or erectile dysfunction was regarded as only for some of the degenerates and could be mentioned only with disdain and hesitation.

An epidemiological study indicates, however, that in recent years, a majority of the males in Japan 40 to 70 years of age have ED and it is said that no less than 1,000 males have the disorder.

In the circumstances, a male who has developed ED starts worrying to a considerable degree. Then the physical contact between the spouses ceases to exist and a good marital relationship breaks down, possibly leading to a divorce. While ED due to age is in most cases inevitable, there were cases where breakups of marital relationships occurred even among elderly couples despite of their emotional bonds founded on a long-standing trust between the spouses.

### SUMMARY OF THE INVENTION

It is an object of the present invention to provide an insertion assisting device that enables a penis, though having a decreased erectile function, to be inserted smoothly into a vagina and dissolves within a few minutes, say, 10 minutes, preferably within a short time period of 3 to 6 minutes of insertion, with the result that the penis is kept in the vagina in a natural state of insertion, thus providing a good physical contact between spouses and maintaining a desirable marital relationship.

Accordingly, the present invention provides an insertion assisting device for inserting a penis into a vagina, comprising: a cylindrical member for covering the penis, said cylindrical member including: a front portion having a round tip at which said front portion closes and tapering in a curve toward said round tip; and a cylindrical portion having a circular opening end, herein said cylindrical member is a hard capsule formed from a soluble material that dissolves in body fluid existing inside the vagina.

The soluble material is preferably a soluble vegetable material.

The soluble vegetable material is preferably a vegetable pulllulan or HPMC (hydroxypropylmethylcellulose).

The soluble material is preferably a soluble animal material.

The soluble animal material is preferably pork gelatin, beef gelatin or a mixture thereof.

The cylindrical member preferably has a layer containing a contraceptive formed on at least a part of its inner surface or its outer surface.

The cylindrical member is preferably a hard capsule formed from a soluble material containing a contraceptive.

Accordingly, the present invention also provides an insertion assisting device for inserting a penis into a vagina, comprising a cylindrical member for covering a penis, the cylindrical member including a cylindrical portion with a front portion tapering in a curve toward its tip at which it closes and an opening end, the cylindrical member being a hard capsule formed from a meltable material that melts at body temperatures of the penis and the vagina.

According to the present invention, the insertion assisting device allows a penis, though having a decreased erectile function due to ED or other disorders, to be smoothly inserted into a vagina and dissolves within a few minutes, say 10 minutes, preferably within a short time period of 3 to 6 minutes of insertion. Thus, the penis can be kept in a natural state of insertion in the vagina, and a good physical contact between spouses and a desirable marital relationship can be maintained.

Further, the use of a naturally occurring material such as a soluble vegetable material as exemplified by a vegetable pulllulan and HPMC (hydroxypropylmethylcellulose) or a soluble animal material as exemplified by pork gelatin and beef gelatin to provide the soluble material that forms the insertion assisting device of the invention ensures an enhanced safety and gives the user a sense of safety so that the user can use the insertion assisting device of the present invention knowing it is safe.

### BRIEF DESCRIPTION OF THE DRAWINGS

Figs. 1A and 1B are a front view and a lateral view as seen from an end, respectively, of a preferred embodiment of the insertion assisting device according to the invention.
Figs. 2A and 2B are a front view and a lateral view as seen from an end, respectively, of another embodiment of the insertion assisting device according to the invention.
Figs. 3A and 3B illustrate a method for attaching the insertion assisting device shown in Figs. 1A and 1B.

An insertion assisting device according to the present invention will be described in detail based on preferred embodiments illustrated in the attached drawings.

### DETAILED DESCRIPTION OF THE INVENTION

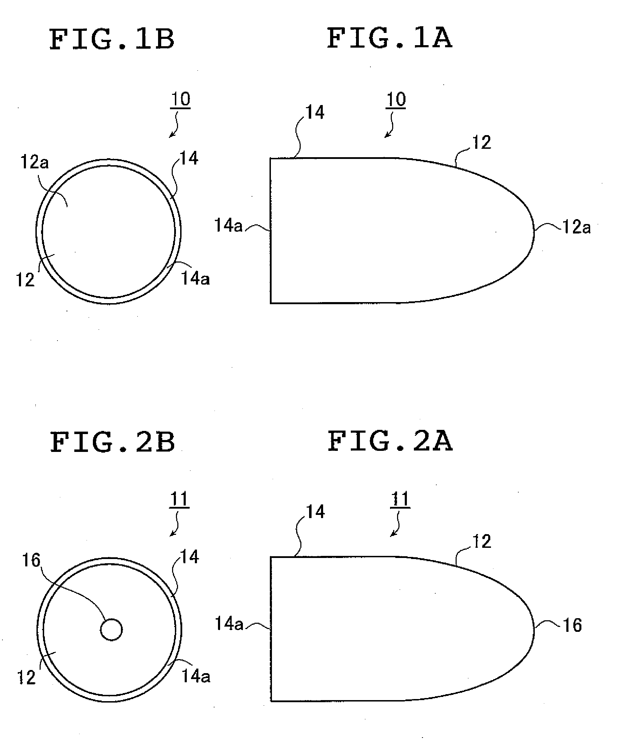

As illustrated in Figs. 1A and 1B, an insertion assisting device 10 is a substantially cylindrical member that allows a penis, even when it is not erect because its erectile function has decreased due to ED or other like disorders, to be inserted smoothly into a vagina and dissolves in body fluid after insertion, preferably in a short period of time, such that the penis is kept in the vagina in a natural state of insertion. The cylindrical member comprises a front portion 12 tapering in a curve toward a round front tip 12a at which it closes and a cylindrical portion 14 terminating with a circular opening end 14a.

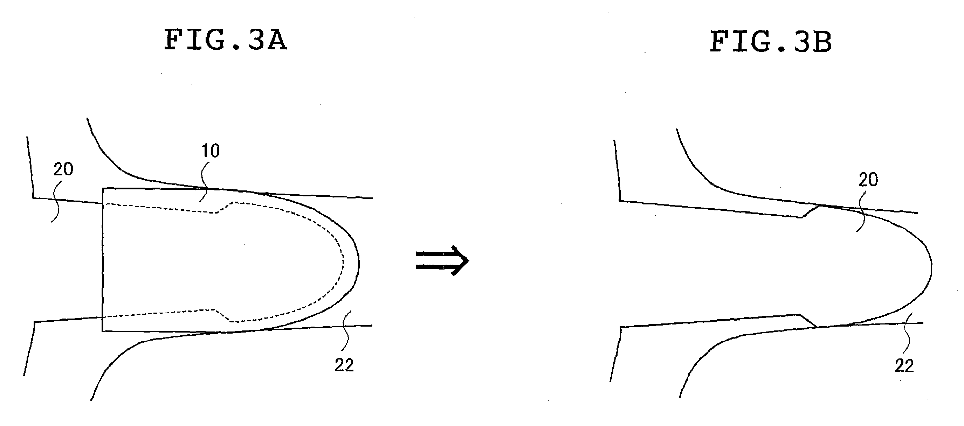

The insertion assisting device 10 according to the present invention dissolves of itself in body fluid in the vagina indicated by 22 in Figs. 3A and 3B as the penis indicated by 20 in Figs. 3A and 3B is held inside the insertion assisting device 10, thereby allowing the penis to remain in the vagina in a natural manner.

The insertion assisting device 10 is required to allow the penis, though failing to erect, to be inserted therein from the opening end 14a- and so cover the penis, and is, in addition, also required to be inserted into the vagina while holding the penis therein. Accordingly, the insertion assisting device 10 must have a sufficient strength to fulfill two functions: a function to hold the penis, though failing to erect, and another function whereby it can be inserted into the vagina as it holds in itself the penis. Stated otherwise, the insertion assisting device 10 needs to have a shape retaining capability to hold the penis therein and maintain its shape.

Another requirement is that the insertion assisting device 10 of the present invention needs to have a sufficient solubility that it is capable of performing a function of dissolving of itself as quickly as possible in body fluid existing inside the vagina, allowing the penis to remain in the vagina 22 in a natural manner. Still another requirement is that the insertion assisting device 10 must be safe: it not only needs to be harmless to the human body, i.e., have no detrimental effects on the human body before and after insertion but also needs to keep the penis as inserted naturally in the vagina and must not allow the user to have an uncomfortable sensation in use.

Accordingly, the insertion assisting device 10 needs to be manufactured using a material having the shape retaining capability, the solubility and the safety. The material used to manufacture the insertion assisting device 10 is not specifically restricted and may be any material, provided that it has the shape retaining capability, the solubility and the safety as described above.

For example, the insertion assisting device 10 dissolves as time passes in the presence of, for example, body water such as body fluid existing inside the vagina or body fluid from the penis. For example, it preferably dissolves within 5 minutes of insertion into the vagina, or more preferably, at least part of it dissolves within 2 minutes of insertion. Accordingly, the soluble insertion assisting device 10 may be such that it melts at a body temperature as well as it dissolves in body water as described above.

According to the invention, the insertion assisting device 10 may be formed of a soluble material such as a soluble animal material as exemplified by gelatins including pork gelatin, beef gelatin, or a mixture thereof, or a soluble vegetable material as exemplified by polysaccharides including pulllulan and cellulose derivatives (cellulose ethers) including HPMC (hydroxypropylmethylcellulose).

According to the invention, the insertion assisting device 10 may be formed in such a manner that a soluble material such as a gelatin, pulllulan, or HPMC in the form of powder is stirred, dissolved, and jelled, whereon it is supplied to a jelly tank; forming pins specially provided for the formation of the insertion assisting device 10 are immersed in the jelly tank to allow a given thickness of the jelly to attach onto the forming pins; and upon drying, the jelly of the given thickness thus formed is detached from each of the forming pins after it has dried, producing the insertion assisting device 10. To manufacture the insertion assisting device 10, a conventional capsule manufacturing machine may be adapted such that it can use the forming pins designed specifically for the formation of the insertion assisting device 10.

The forming pins or the surface of the jelly (a soluble material in the form of jelly) attached to the forming pins may be coated with a wax of, for example, a monosaccharide, a polymer, or fat to manufacture the insertion assisting device 10, or any of these coating materials themselves may be used to form the insertion assisting device 10. Alternatively, the insertion assisting device 10 may be produced by first forming the soluble material into a film or by forming any of said coating materials into a film.

Using the wax as described above in the insertion assisting device 10, an advantage may be had that the wax acts as a lubricant as the surface melts at body temperatures of the vagina and the penis as described earlier, thus making it easier to insert the penis into the insertion assisting device 10 and insert the insertion assisting device 10 with the penis held therein into the vagina.

Materials that may be used to manufacture the insertion assisting device 10 include all the materials used in the known capsules as exemplified by capsules used for oral administration of medication that contain filled therein medicines for internal application (i.e., capsules for drugs), food capsules used for other purposes than for drugs to contain foods such as dietary supplements and health food products, and capsules used in a wide variety of fields including those used for quasi-drugs, drugs for animals, agricultural chemicals, health food products, general processed foods, and toys. Materials used for hard microcapsules in particular are preferred materials for the insertion assisting device 10.

For example, soluble animal materials such as gelatins including pork gelatin, beef gelatin, or a mixture thereof; soluble vegetable materials as exemplified by polysaccharides such as pulllulan and cellulose derivatives (cellulose ethers) including HPMC (hydroxypropylmethylcellulose), MC (methylcellulose), CMC (carboxymethylcellulose), CMC Na (sodium carboxymethylcellulose), and CMC Ca (carboxymethylcellulose calcium) are more preferably used, and, in particular, soluble animal materials such as gelatins including pork gelatin, beef gelatin, or a mixture thereof, and soluble vegetable materials such as pulllulan and HPMC (hydroxypropylmethylcellulose) are still more preferably used for the insertion assisting device 10.

Gelatins are produced from collagen, which is contained in hides, bones and tendons of animals and is a major component of the intercellular substances in animal connective tissue. Gelatins are obtained by heating collagen with water for long hours to make them water soluble. In short, gelatins are collagen made soluble by thermal denaturation.

Gelatins, and in particular gelatins such as pork gelatin, beef gelatin, or a mixture thereof, are animal proteins that are harmless to the human body, not causing unnatural sensation, and easily absorbed in the human body. Further, gelatins solate or gelate, form a hard film on themselves as they dry, have a low oxygen and water permeability and, in addition, are easily available and sold at low costs. Because of these features, gelatins are widely used as materials for capsules.

Pullulan is a soluble vegetable material that is safe to use and has excellent functions. It consists of soluble polysaccharides produced from a raw material being corn starch, most preferably non-genetically modified corn starch, by fermentation using a black yeast (Aureobasidium pullulans). In the present invention, the term polysaccharides refer to all the carboydrates that produce monosaccharides having 2 or more molecules by hydrolysis and include olygomers such as disaccharides, trisaccharides, and tetrasaccharides. The polysaccharides that may be used in the present invention include starches, glycogen, inulin, lichenin, celluloses, chitins, hemicelluloses (e.g., agar), galactose, and pectins.

Pullulan, of which the safety has been proven by such tests as acute toxicity tests, subacute toxicity tests, chronic toxicity tests, and mutagenesis tests, is widely used in Japan as an additive without restriction of use for foods, drugs, and cosmetic products and is also widely used overseas.

HPMC is a designated food additive in Japan, the United States, and European Union and is used as base material of capsules for foods with health claims and as a tablet binder. Likewise, MC, CMC, CMC Na and CMC Ca are used in the United States and Europe as general food additives or as materials of capsule bases for dietary supplements, as well as tablet binders and tablet coatings.

Thus, the use of gelatins, which are animal materials, such as pork gelatin, beef gelatin or a mixture thereof or vegetable materials such as pullulan and HPMC in the insertion assisting device 10 of the present invention results in the manufacture of the insertion assisting device 10 that has no harmful effects upon the human body and possesses shape retaining capability, solubility and safety at the same time.

As a matter of course, when manufacturing the insertion assisting device 10 using a soluble material such as a gelatin, pullulan, and HPMC, the soluble material may be supplemented with known additives such as gelators and auxiliary substances as exemplified by carrageenan, potassium chloride, sodium lauryl sulfate, sucrose fatty acid ester, and soy lecithin.

While preferably the insertion assisting device 10 is relatively thick for the shape retaining capability described above on one hand, it is preferably relatively thin to be quickly soluble on the other. Accordingly, no specific restriction is set to the thickness of the insertion assisting device 10 and it may be determined as appropriate seeking a balance between shape retaining capability and solubility as required. Thus, the thickness may be set to 0.08 mm (80 µm) to 1 mm, preferably 0.08 mm (80 µm) to 0.3 mm (300 µm), more preferably 0.08 mm (80 µm) to 0.15 mm (150 µm), and still more preferably 0.09 mm (90 µm) to 0.12 mm (120 µm).

The inner diameter of the insertion assisting device 10 is not specifically restricted, provided that the insertion assisting device 10 can accommodate the penis therein and that its external shape allows it to be inserted into the vagina. Preferably, the inner diameter may be set for example to 30 mm to 45 mm and, more preferably, 33 mm to 38 mm.

The length of the insertion assisting device 10 is not specifically restricted, provided that the insertion assisting device 10 can accommodate the penis therein and that its external shape allows it to be inserted into the vagina. Preferably, the length may be set for example to 50 mm to 100 mm and, more preferably, 50 mm to 80 mm.

The shape and the dimensions of the front portion 12 of the insertion assisting device 10 are not specifically restricted and may be determined as appropriate, provided that the front portion 12 can accommodate the front part of the penis therein, that its external shape allows the insertion assisting device 10 to be inserted into the vagina, and that the front portion 12 tapers in a curve toward the tip 12a at which it closes. Preferably, the length of the front portion 12, for example, may be set to 30 mm to 45 mm, and more preferably 36 mm to 38 mm. The shape of the front portion near the tip may assume a smooth convex form and be, for example, spherical, ellipsoidal, or paraboloidal.

While the insertion assisting device 10 described above has the front portion 12 that closes at the tip, it is not specifically restricted to such configuration. As exemplified by an insertion assisting device 11 illustrated in Fig. 2A and Fig. 2B, the front portion 12 may have an aperture 16 formed at its tip. While the illustrated example has the aperture 16 located at the tip taking the strength and the shape retaining capability of the insertion assisting device 11 as well as other factors into consideration, the location of the aperture 16 is not specifically restricted and it may be located in any position as desired, provided that the strength and shape retaining capability of the insertion assisting device 11 are ensured. For example, the aperture 16 may be provided somewhere in the front portion 12 or in the cylindrical portion 14, preferably in the front portion 12, instead of or in addition to the one located at the tip of the front portion 12.

Thus, the insertion assisting device 11 illustrated in Fig. 2A and Fig. 2B, provided with the aperture 16 at the tip of the front portion 12 or in other positions, allows the penis to be easily inserted from the opening end 14a, facilitating the attachment of the insertion assisting device 11 onto the penis. In particular, when there is a substantial difference in the amount of body fluid between vagina and penis, body fluid can be supplied from one of the vagina and the penis with a relatively larger amount of body fluid to the other, as the insertion assisting device holding the penis therein is kept inserted in the vagina, thus promoting the dissolution of the insertion assisting device 11 from the inside and the outside thereof simultaneously.

The insertion assisting devices 10 and 11 of the invention may include a layer containing a contraceptive at least on part of the inner or outer surface thereof. Alternatively, the insertion assisting devices 10 and 11 may be formed by forming a soluble material containing a contraceptive into a hard capsule. In the case where a layer containing a contraceptive is formed at least on part of the inner or outer surface of the insertion assisting devices 10 and 11, the layer containing a contraceptive may be provided on any of the surfaces. However, it is preferable that the inner surface is provided with a layer containing a male contraceptive whereas the outer surface is provided with a layer containing a female contraceptive. In this case, only one of said layers containing a contraceptive may be provided or both layers may be provided. When a contraceptive is included in the soluble material used, it may be a male contraceptive or a female contraceptive.

No specific restriction is set to the contraceptives used in the present invention and those selected from a variety of known contraceptives may be used in the present invention.

The insertion assisting device according to the invention has been described above in respect of its basic configuration. Now its operation and use will be described.

Fig. 3A and Fig. 3B illustrate the way the insertion assisting device shown in Fig. 1A and 1B is attached. First, as illustrated in Fig. 3A, a penis 20, though failing to erect, is inserted into the insertion assisting device 10 from its opening end 14a. In this example, the penis 20 is introduced in the insertion assisting device until the front end of the penis 20 reaches the tip 12a of the front portion 12. Thus, the insertion assisting device 10 is attached to the penis 20, preferably such that it covers substantially the whole penis 20.

Next, the insertion assisting device 10 that has been attached to the penis 20 as illustrated in Fig. 3A is led to a vagina 22 by, for example, holding the insertion assisting device 10 in a hand. When the insertion assisting device 10 with the penis 20 held therein has been fully introduced into the vagina 22, the introduction is stopped.

Thereafter, the insertion assisting device 10 now located between the vagina 22 and the penis 20 dissolves of itself in the presence of body fluid in the vagina 22 and possibly of body fluid from the penis 20, or melts at a body temperature of the vagina 22 and/or the penis 20, or for other causes. Thus, the penis 20 is allowed to remain inserted in the vagina 22 as illustrated in Fig. 3B.

Thus, the insertion assisting device allows the penis, though suffering a decreased erectile function or erectile incompetence due to ED or other disorders, to be inserted smoothly into the vagina and dissolves within a few minutes, say 10 minutes, preferably within a short time period of 3 to 6 minutes of insertion. Accordingly, the penis can be kept inserted in the vagina in a natural manner. Thus, a good physical contact between spouses is maintained and a good marital relationship continues. Accordingly, even an elderly couple can acknowledge not only their emotional bond based on their long-standing relationship of mutual trust but also their physical bond, keeping them on good terms with each other and ensuring a happy marital life thereafter. This avoids the possibility that a disorder such as ED should cause loss of physical contact and a physical bond between spouses and that a good marital relationship should come to an end, and which helps eliminate the occurrence of a crisis that may lead to a divorce or the approach of a breakup.

Now, a manufacturing method of the insertion assisting device according to the invention will be described.

Prepared as material is a soluble material such as gelatin, HPMC, or pullulan added, when necessary, with a gelator and/or other auxiliary substances.

The soluble material is directly put, while being stirred, in a solution tank in which water with a given temperature has been poured.

Then, when dissolution has been completed, the heat of the solution is roughly removed and the temperature thereof is lowered to short of a temperature at which it will harden.

Next, the jelly thus obtained is supplied to a capsule manufacturing machine adapted to suit the use in the present invention, and forming pins dedicated to the manufacture of the insertion assisting device of the invention are immersed in a jelly tank. Subsequently the jelly is dried up in a temperature- and humidity-controlled drying room wherein the air volume used is set to a given level.

Finally, a device, now dry and obtained in the form of a capsule on each of the forming pins, is withdrawn therefrom and cut to a given size to produce an insertion assisting device of the present invention.

### EXAMPLES

### [Example 1]

The insertion assisting device of the invention will now be described specifically based on examples below.

First, gelatin (of pork and beef origin), HPMC, and pullulan all in powder form were prepared as materials. These powdered materials were each added with 0.35 % to 1 % of carrageenan as gelator and supplemented with 0.35 % to 0.50 % of potassium chloride as auxiliary substance.

The soluble materials thus prepared were dissolved as they were directly put, while being stirred, in solution tanks in which 50°C to 70°C water has been poured. The dissolution temperature was 50°C to 70°C and the dissolution time was 15 minutes to 20 minutes.

Then, when dissolution has been completed, the heat of the solution was roughly removed and the temperature of the solution was lowered to about 40°C at which the solution would stop short of hardening. At this time, the pH of the solution was about 6.0 and its viscosity was 40 mp to 50 mp.

Next, the jelly thus obtained was supplied to a capsule manufacturing machine adapted to suit the purpose. Then forming pins dedicated to the manufacture of the insertion assisting device of the invention were immersed in jelly tanks. Subsequently the jelly was dried for 30 minutes to 60 minutes in a temperature- and humidity-controlled drying room wherein the air volume used was set to a given level.

Finally, a device formed into a dried capsule on each of the forming pins was withdrawn therefrom and cut to a given size to produce the insertion assisting device 10 of the present invention as illustrated in Figs. 1A and 1B.

The insertion assisting device 10 of the present invention in the form of a hard capsule thus obtained measured 35 mm in diameter, 60 mm in length, and 80 µm to 130 µm in thickness.

A disintegration test was conducted on samples of the insertion assisting device 10 of the present invention in the form of a hard capsule.

The test used a 2-basket disintegration tester manufactured by Toyama Sangyo Co., Ltd. with two baskets to receive samples.

Prepared for the test were three different disintegration liquids: physiological saline solution (of near neutrality), physiological saline solution (pH 4), and a disintegration test liquid No. 1 (pH 1.5). The physiological saline solution used was a 0.9 % physiological saline solution.

Three different samples were used: an insertion assisting device of gelatin origin, an insertion assisting device of HPMC origin, and an insertion assisting device of pullulan origin. The insertion assisting device of gelatin origin had a water content of about 15 % whereas the insertion assisting devices of HPMC origin and of pullulan origin both had a 2 % to 4 % water content.

The test was conducted as follows.

The three different disintegration liquids were poured into the 2-basket disintegration tester, one disintegration liquid at a time. A same disintegration liquid was used in the two baskets, with 1000 ml poured in each of them. Then the temperature was adjusted to 36°C to 37°C to conduct a preliminary test, which indicated that almost no change was observed at 36°C or 37°C. The disintegration tester was of convection type whereby the warm water was stirred evenly.

Samples were each immersed in the baskets of the disintegration tester to measure the disintegration time.

The results are shown in Tables 1, 2 and 3.

**[Table 1]**

| Gelatin origin | Minimum | Maximum | Average |
|---|---|---|---|
| Normal saline (near neutrality) | 1 min. 30 s | 4 min. 50 s 3 | min. 20 s |
| Normal saline (pH 4) | 1 min. 40 s | 4 min. 30 s | 2 min. 40 s |
| Disintegration test liquid No. 1 (pH 1.5) | 1 min. 00 s | 3 min. 20 s | 1 min. 50 s |

**[Table 2]**

| HPMC origin | Minimum | Maximum | Average |
|---|---|---|---|
| Normal saline (near neutrality) | 1 min. 40 s | 4 min. 40 s | 3 min. 10 s |
| Normal saline (pH 4) | 1 min. 30 s | 4 min. 20 s | 2 min. 30 s |
| Disintegration test liquid No. 1 (pH 1.5) | 1 min. 10 s | 3 min. 10 s | 1 min. 40 s |

**[Table 3]**

| Pullulan origin | Minimum | Maximum | Average |
|---|---|---|---|
| Normal saline (near neutrality) | 1 min. 30 s | 4 min. 50 s | 3 min. 20 s |
| Normal saline (pH 4) | 1 min. 30 s | 4 min. 20 s | 2 min. 30 s |
| Disintegration test liquid No. 1 (pH 1.5) | 1 min. 00 s | 3 min. 00 s | 1 min. 40 s |

As shown in Tables 1, 2 and 3, all the samples of any of the three origins dissolved within 10 minutes of the start of the test. The disintegration of the test samples occurred in a favorable manner under all the test conditions used. In the test, the disintegration time was the time it took for the samples to lose their shapes.

From the above test results, it appears that there is no substantial difference in disintegration time between the samples of gelatin origin, HPMC origin and pullulan origin.

It is observed that the sample disintegration time tends to shorten, i.e., the disintegration tends to progress more quickly, though the differences are small, as the pH of the disintegration liquid lowers i.e., as the acidity increases.

It appears from the above that the disintegration of samples is affected by the composition (pH) of the disintegration liquid and the intensity of physical impacts.

The results of Example 1 clearly show that the insertion assisting device of the present invention dissolves within 5 minutes, i.e., within 10 minutes in body fluid and the advantageous effects produced by the present invention are apparent.

### [Example 2]

Only powdered gelatin (of pork origin) was prepared as a material to produce samples of the insertion assisting device 10 of the present invention illustrated in Figs. 1A and 1B, similarly to Example 1, each with different dimensions as shown in Table 4.

Test samples 1, 2, and 3 of the insertion assisting device 10 of the present invention with different dimensions as shown in Table 4 were used to conduct a disintegration test similarly to Example 1.

The test results are shown in Table 5, 6, and 7.

**[Table 4]**

| Kinds | Diameter (mm) | Length (mm) | Thickness (µm) |
|---|---|---|---|
| Sample 1 | 30 | 50 | 100 to 150 |
| Sample 2 | 35 | 60 | 100 to 150 |
| Sample 3 | 40 | 70 | 100 to 150 |

**[Table 5]**

| Sample 1 | Minimum | Maximum | Average |
|---|---|---|---|
| Physiological saline (near neutrality) | 3 min. 30 s | 6 min. 50 s | 5 min. 10 s |
| Physiological saline (pH 4) | 3 min. 40 s | 6 min. 30 s | 5 min. 05 s |
| Disintegration test liquid No. 1 (pH 1.5) | 3 min. 00 s | 7 min. 20 s | 5 min. 10 s |

**[Table 6]**

| Sample 2 | Minimum | Maximum | Average |
|---|---|---|---|
| Physiological saline (near neutrality) | 3 min. 40 s | 6 min. 40 s | 5 min. 10 s |
| Physiological saline (pH 4) | 3 min. 30 s | 6 min. 20 s | 4 min. 55 s |
| Disintegration test liquid No. 1 (pH 1.5) | 3 min. 10 s | 6 min. 10 s | 4 min. 40 s |

**[Table 7]**

| Sample 3 | Minimum | Maximum | Average |
|---|---|---|---|
| Physiological saline (near neutrality) | 3 min . 40 s | 6 min. 50 s | 5 min. 15 s |
| Physiological saline (pH 4) | 3 min. 30 s | 6 min. 30 s | 5 min. 00 s |
| Disintegration test liquid No. 1 (pH 1.5) | 3 min. 20 s | 6 min. 20 s | 4 min. 50 s |

As is clear from Table 5, 6, and 7, all the samples dissolved within 10 minutes of the start of the test in all the three kinds of disintegration liquids. The disintegration of the test samples occurred in a favorable manner under all the test conditions used. In the test, the disintegration time was the time it took for the samples to lose their shapes.

From the above, no substantial difference is apparently observed in disintegration time between samples of pork origin with different dimensions.

The test results in Examples 1 and 2 indicate that a difference in disintegration time due to the thickness of the samples is observed such that the disintegration time grows longer i.e., the disintegration occurs more slowly as the thickness grows greater.

Further, as in the case of Example 1, a tendency is observed that the sample disintegration time shortens, i.e., the disintegration progresses more quickly, though the differences are small, as the pH of the disintegration liquid lowers i.e., as the acidity increases.

It appears from the above that the disintegration of samples is affected by the composition (pH) of the disintegration liquid and the intensity of physical impacts.

The results of Example 2 also clearly show that the insertion assisting device of the present invention dissolves within 5 minutes, i.e., within 10 minutes in body fluid and the advantageous effects produced by the present invention are apparent.

While the insertion assisting device according to the present invention has been described in detail by means of specific embodiments, it is to be understood that the present embodiments are illustrative and not restrictive and that numerous changes and modifications may be made therein without departing from the spirit and scope of the invention.

For example, the insertion assisting device of the present invention may be formed using a carrier or a base used for suppositories. For example, materials that melt or soften at body temperature may be used as such carrier or base and are exemplified by cacao butter, glycerogelatin, sodium stearate, and propylene glycol monostearate. Such carrier or base used for suppositories may be used without modification to form the insertion assisting device or may be used as coating material applied to the surface of the insertion assisting device that is manufactured using, for example, any of the polysaccharides and monosaccharides described above.

## Claims

1. An insertion assisting device for inserting a penis into a vagina, comprising:
a cylindrical member for covering the penis, said cylindrical member including: a front portion having a round tip at which said front portion closes and tapering in a curve toward said round tip; and a cylindrical portion having a circular opening end,
wherein said cylindrical member is a hard capsule formed from a soluble material that dissolves in body fluid existing inside the vagina.

2. The insertion assisting device of Claim 1, wherein said soluble material is a soluble vegetable material.

3. The insertion assisting device of Claim 2, wherein said soluble vegetable material is vegetable pullulan or HPMC (hydroxypropylmethylcellulose).

4. The insertion assisting device of Claim 1, wherein said soluble material is a soluble animal material.

5. The insertion assisting device of Claim 4, wherein said soluble animal material is pork gelatin, beef gelatin or a mixture thereof.

6. The insertion assisting device of any one of Claims 1 to 5, wherein said cylindrical member has a layer containing a contraceptive formed on at least a part of its inner surface or its outer surface.

7. The insertion assisting device of any one of Claims 1 to 5, wherein said cylindrical member is a hard capsule formed from a soluble material containing a contraceptive.
